# EUROPÄISCHE PATENTSCHRIFT

(11) **EP 4 474 042 B1**
(45) Veröffentlichungstag und Bekanntmachung des Hinweises auf die Patenterteilung: **31.12.2025**
(21) Anmeldenummer: 23177510.7
(22) Anmeldetag: 06.06.2023
(51) Int. Cl.: B01F 31/441, B01F 33/501, B01F 35/32, B01F 35/71

(54) **MISCHSYSTEM MIT SEITLICHEM AMPULLENBRECHER**
MIXING SYSTEM WITH SIDE AMPOULE BREAKER
SYSTÈME DE MÉLANGE AVEC BRISE-AMPOULE LATÉRAL

(43) Veröffentlichungstag der Anmeldung: 11.12.2024
(73) Patentinhaber: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(56) Entgegenhaltungen:
- EP-A1- 2 987 765
- EP-A1- 4 169 608
- EP-B1- 2 404 864
- EP-B1- 3 093 067
- WO-A1-2023/047359

## Beschreibung

### GEBIET DER ERFINDUNG

Die vorliegende Erfindung betrifft das Gebiet der Medizintechnik, insbesondere Vorrichtungen für die Zubereitung von Knochenzement. Insbesondere werden Vorrichtungen zum Öffnen von Glasampullen beschrieben, welche zur Abgabe einer Flüssigkeit aus solchen Glasampullen geeignet sind. Beispielsweise können durch die hierin beschriebenen Vorrichtungen eine Monomerflüssigkeit an eine Pulverkomponente zur Herstellung eines Polymethylmethacrylat-Knochenzements abgegeben werden. Die hierin beschriebenen Vorrichtungen können darüber hinaus auch allgemein zum Lagern und Öffnen von Glasampullen und zur Abgabe einer darin enthaltenen Flüssigkeit dienen.

### TECHNISCHER HINTERGRUND

Polymethylmethacrylat-(PMMA)-Knochenzemente gehen auf die grundlegenden Arbeiten von Charnley zurück. PMMA-Knochenzemente beinhalten regelmäßig eine flüssige Monomerkomponente und einer Pulverkomponente. Die Monomerkomponente enthält häufig das Monomer Methylmethacrylat und einen darin gelösten Aktivator (üblicherweise N,N-Dimethyl-p-toluidin). Die Pulverkomponente, auch als Knochenzementpulver bezeichnet, weist üblicherweise ein oder mehrere Polymere, die auf Basis von Methylmethacrylat und Comonomeren, wie beispielsweise Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs bis dieser erstarrt.

Polymethylmethacrylat-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können. Zur Vermeidung von Lufteinschlüssen im Knochenzementteig wurden eine Vielzahl von Vakuum-Zementiersystemen beschrieben, von denen exemplarisch folgende genannt sind: US6,033,105A, US5,624,184A, US4,671,263A, US4,973,168A, US5,100,241A, WO99/67015A1, EP1 020 167A2, US5,586,821A, EP1 016 452A2, DE36 40 279A1, WO94/26403A1, EP1 005 901A2, und US5,344,232A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die bevorzugt erst unmittelbar vor der Zementapplikation im Zementiersystem miteinander vermischt werden können. Solche geschlossenen Full-Prepacked-Mischsysteme wurden in den Patenten EP0380867B1, EP0796653B1, EP0692229B1, DE102009031178B1, US5997544B1, US6709149B1, DE69812726B1 und US5588745B1, WO9416951A1, DE19718648A1, EP1741413B1, EP3054880B1, DE102009031178B3, US8662736B2 und EP3093067B1, das eine Vorrichtung nach dem Oberbegriff des Anspruchs 1 offenbart, beschrieben.

Im EP1741413B1 wird ein Mischsystem beschrieben, das aus zwei miteinander verschraubten Kartuschen besteht. Zementpulver befindet sich im Innenraum der Kartuschen. Die untere Kartusche enthält einen Hohldorn oder zwei Hohldorne, die mit dem Innenraum der zweiten Kartusche flüssigkeitsdurchlässig verbunden sind. Die Spitzen der Hohldorne sind mit einer durchstechbaren Gummikappe verschlossen. Diese dürfen während der Lagerung und des Transports nicht abfallen, weil ansonsten das Knochenzementpulver aus den Kartuschen durch die Hohldorne austreten kann. Es befinden sich ein oder zwei mit Monomerflüssigkeit befüllte Beutel auf einem Schienensystem an der zweiten Kartusche, wobei die Beutel gegen die Hohldorne geschoben werden können und die Hohldorne zuerst die Gummikappen und dann die Beutel perforieren. Durch Anlegen von Vakuum an die beiden Kartuschen wird die Monomerflüssigkeit aus den Beuteln in den Innenraum der beiden Kartuschen gesaugt. Danach werden mit einem Mischer die beiden Knochenzementkomponenten gemischt und danach wird der Austragskolben in der unteren Kartusche gelöst und dieser sammelt den gebildeten Knochenzementteig in der oberen Kartusche, wobei der Austragskolben sich aus der unteren Kartusche in die obere Kartusche bewegt. Die untere Kartusche wird zusammen mit den entleerten Beuteln von der oberen Kartusche abgeschraubt. Danach steht die obere Kartusche zur Applikation des Knochenzementteigs bereit. Monomerflüssigkeitsbeutel sind prinzipiell geeignete Behälter zur Aufbewahrung von Monomerflüssigkeit. Sie sind jedoch mit einer Reihe von Problemen verbunden. Die Monomerflüssigkeitsbeutel sind in ihrer Herstellung und bei der aseptischen Befüllung sehr aufwendig. Weiterhin ist die Abdichtung der perforierten Beutel im Bereich der Hohldorne schwierig während des Monomerflüssigkeitstransfers. Der vollständige Transfer der Monomerflüssigkeit aus den Beuteln gelingt nicht immer vollständig, weil die Beutel durch die Vakuumeinwirkung kollabieren. Es können dabei Reste der Monomerflüssigkeit in den kollabierten Beuteln verbleiben.

Die Monomerflüssigkeit von PMMA-Knochenzement lässt sich beispielsweise in Glasampullen, sowie in Kunststoffverbundbeuteln, als auch in metallisierten Kunststoffverbundbeuteln lagern. Zur Entnahme der Monomerflüssigkeit aus einer Glasampulle kann üblicherweise der Ampullenkopf vom Ampullenkörper abgetrennt und entfernt werden. Vorrichtungen zum Öffnen von Ampullen sind in den Schriften DE19532015A1, WO9718031A1, EP1031333A1 und WO2010012114A1 offenbart.

EP2404864B1 offenbart eine Vorrichtung, bei der eine Ampulle in einem Ampullenhalter angeordnet ist und wobei im Bereich des Ampullenhalses der Ampullenhalter elastisch deformierbar ist. Im Bereich des Ampullenkopfs und des Ampullenkörpers ist der Ampullenhalter nicht deformierbar. Durch Knicken des Ampullenhalters über den elastisch deformierbaren Abschnitt des Ampullenhalters, der eine Scharnierfunktion hat, kann der Ampullenkopf gegen die nicht deformierbare Wandung des Ampullenhalters gedrückt werden. Dadurch kann der Ampullenkopf abbrechen und auf ein Sieb fallen. Die Monomerflüssigkeit kann danach aus der geöffneten Ampulle durch das Sieb fließen. Für die Funktion der dort beschriebenen Vorrichtung ist es vorteilhaft, dass diese mit einem mit Monomerflüssigkeit zu versorgenden Mischsystem mechanisch stabil verbindbar ist, weil eine erhebliche Zugbelastung beim Abknickvorgang an der Befestigung zum Mischsystem auftreten kann.

### BEVORZUGTE AUSFÜHRUNGSFORMEN

Die Aufgabe der vorliegenden Erfindung besteht darin, eines oder mehrere der oben geschilderten und weitere Probleme des Stands der Technik zu lösen. Beispielsweise ermöglicht die Erfindung eine einfache und sichere Abgabe einer Flüssigkeit aus einer Glasampulle an eine Kartusche mit einer Pulverkomponente. Weiterhin liefert die vorliegende Offenbarung ein Verfahren zur Abgabe einer Flüssigkeit aus einer Glasampulle an eine Kartusche mit einer Pulverkomponente.

Diese Aufgaben werden gelöst durch die hierin beschriebenen Verfahren und Vorrichtungen, genauer gesagt, durch die Vorrichtungen, die in den Patentansprüchen beschrieben sind.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Verfahren beschrieben werden, auch für die hierin beschriebenen Erzeugnisse und Vorrichtungen anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein erster Aspekt betrifft eine Vorrichtung zur Herstellung eines Knochenzements, aufweisend
- einen Ampullenbrecher mit einem ersten Gehäuse, wobei das erste Gehäuse einen ersten Innenraum aufweist,
- eine in dem ersten Gehäuse angeordnete Halterung zur Aufnahme einer Glasampulle,
- ein in dem ersten Gehäuse angeordnetes Aufbrechelement, das zum Aufbrechen einer in der Halterung aufnehmbaren Glasampulle ausgebildet und eingerichtet ist,
- ein in dem ersten Gehäuse angeordneter Sammelbereich, der zur Aufnahme einer Flüssigkeit aus einer aufgebrochenen Glasampulle ausgebildet und eingerichtet ist,
- eine Kartusche, die zur Aufnahme einer Pulverkomponente eines Knochenzements eingerichtet ist, wobei die Kartusche ein zweites Gehäuse mit einem zweiten Innenraum aufweist,
- einen fluidleitenden Kanal zur Abgabe einer Flüssigkeit aus dem Sammelbereich in den zweiten Innenraum, wobei der fluidleitende Kanal angeordnet und aufgrund eines geringen Innendurchmessers eingerichtet ist, die

Flüssigkeit, durch die Ausbildung eines Meniskus der in dem Kanal aufgenommenen Flüssigkeit, in dem Sammelbereich zurückzuhalten, und die Flüssigkeit an den zweiten Innenraum abzugeben, sobald zwischen dem Sammelbereich und dem zweiten Innenraum ein Druckunterschied anliegt.

Die Vorrichtung weist einen Ampullenbrecher auf. Der Ampullenbrecher bildet bevorzugt eine von mindestens zwei Einheiten innerhalb der erfindungsgemäßen Vorrichtung. Der Ampullenbrecher ist zur Aufnahme einer Glasampulle mit einer Flüssigkeit eingerichtet.

Der Ampullenbrecher weist ein erstes Gehäuse auf, welches einen ersten Innenraum enthält. Innerhalb des ersten Gehäuses ist eine Halterung zur Aufnahme einer Glasampulle angeordnet. Weiterhin weist der Ampullenbrecher ein Aufbrechelement auf, welches zum Aufbrechen einer in der Halterung aufnehmbaren Glasampulle ausgebildet und eingerichtet ist. Wie nachfolgend ausführlicher dargestellt ist, kann das Aufbrechelement in unterschiedlicher Weise ausgebildet sein. Beispielsweise kann das Aufbrechelement einen Kolben und einen Vorsprung aufweisen, wobei eine in der Halterung aufnehmbare Glasampulle mithilfe des Kolbens gegen den Vorsprung gedrückt und dadurch aufgebrochen werden kann. Das Aufbrechelement kann auch eine Taste aufweisen, welche von außen in das erste Gehäuse gedrückt werden kann, um dadurch eine in der Halterung aufnehmbare Glasampulle aufzubrechen. Das Aufbrechelement kann auch als biegsamer Teil des Gehäuses ausgestaltet sein, wobei durch Biegen des Gehäuses eine darin aufnehmbare Glasampulle aufgebrochen werden kann. Das Aufbrechelement kann auch andere im Fachgebiet bekannte Mechanismen zum Aufbrechen einer Glasampulle aufweisen. Gegebenenfalls können mehrere Aufbrechelemente in der Vorrichtung vorgesehen sein, beispielsweise zwei Paare von Kolben und Vorsprüngen, zwei der oben genannten Tasten, oder zwei biegsame Gehäuseabschnitte. Die Taste kann ein elastisches Material aufweisen, um nach dem Eindrücken in das erste Gehäuse selbsttätig in eine ursprüngliche Anordnung der Taste zurückzukehren.

In einigen Ausführungsformen ist der Ampullenbrecher derart stabil mit der Kartusche verbindbar, dass die Betätigung des Aufbrechelements nicht die fluidleitende Verbindung zwischen Ampullenbrecher und Kartusche beeinträchtigt. Hierdurch kann sichergestellt werden, dass mithilfe des Kanals eine im Wesentlichen vollständige Überführung einer Flüssigkeit aus dem Ampullenbrecher in die Kartusche erfolgen kann.

Die Vorrichtung weist weiterhin eine Kartusche auf, die zur Aufnahme einer Pulverkomponente eines Knochenzements eingerichtet ist. Die Kartusche weist ein zweites Gehäuse mit einem zweiten Innenraum auf. Die Kartusche kann einen Verschluss aufweisen, welcher den zweiten Innenraum verschließt. Der Verschluss kann kraftschlüssig oder formschlüssig mit dem zweiten Gehäuse verbunden sein. Der Verschluss kann lösbar mit dem zweiten Gehäuse verbunden sein. Der Verschluss kann beispielsweise ein Gewinde oder einen Schnappverschluss aufweisen. Der Verschluss kann, wie nachfolgend ausführlicher dargestellt, ein Ventil und/oder einen Anschluss für eine Vakuumquelle aufweisen.

Das zweite Gehäuse kann einteilig oder mehrteilig ausgebildet sein. Wenn das zweite Gehäuse mehrteilig ausgebildet ist, kann zwischen zwei Gehäuseteilen des zweiten Gehäuses ein Verbindungselement angeordnet sein, welches die zwei Gehäuseteilen des zweiten Gehäuses miteinander verbindet. Bevorzugt verbindet das Verbindungselement die zwei Gehäuseteilen des zweiten Gehäuses lösbar miteinander, beispielsweise kraftschlüssig oder formschlüssig. Das Verbindungselement kann beispielsweise ein Gewinde oder einen Schnappverschluss aufweisen.

Der Verschluss kann eine Durchführung für einen Mischstab aufweisen.

Das erste Gehäuse und/oder das zweite Gehäuse können transparent oder transluzent sein. Dies kann die visuelle Verfolgung der Bewegung einer Flüssigkeit innerhalb der Vorrichtung ermöglichen.

Die Vorrichtung kann einen von außen bedienbaren Mischer aufweisen. Der Mischer kann in und/oder an der Kartusche angeordnet sein. Der Mischer kann einen Griff, einen Mischstab und eine Scheibe aufweisen, wobei die Scheibe innerhalb des zweiten Gehäuses angeordnet sein kann. Der Mischer kann zur Vermischung einer Flüssigkeit und eines Pulvers ausgebildet und eingerichtet sein.

Die Kartusche kann einen Austragskolben aufweisen oder mit einem Austragskolben verbindbar sein. Der Austragskolben kann beweglich in dem zweiten Gehäuse angeordnet sein. Der Austragskolben kann zur Abgabe eines Knochenzements aus der Kartusche, beispielsweise eines PMMA-Knochenzements, ausgebildet und eingerichtet sein.

Die Vorrichtung weist einen Kanal auf, welcher den ersten Innenraum des Ampullenbrechers fluidleitend mit dem zweiten Innenraum der Kartusche verbindet. Der Kanal weist bevorzugt einen Einlass auf, welcher den ersten Innenraum des Ampullenbrechers, insbesondere den Sammelbereich, mit dem Kanal verbindet. Der Kanal weist bevorzugt einen Auslass auf, welcher den Kanal mit dem zweiten Innenraum der Kartusche verbindet. Der Kanal weist bevorzugt eine Mittelachse M auf, welche durch die kürzeste Verbindung des Mittelpunkts des Einlass mit dem Mittelpunkt des Auslass gebildet wird, und der beabsichtigten Fließrichtung einer Flüssigkeit durch den Kanal entspricht. Diese Mittelachse M kann einen Winkel α mit einer Außenwand der Kartusche bilden. Der Winkel α kann bevorzugt 60° bis 90° betragen, beispielsweise 60° bis 90°, 70° bis 90°, 80° bis 90°, 60° bis 80°, 60° bis 70°, oder 70° bis 80°. Demnach kann der fluidleitende Kanal bevorzugt in einem Winkel α von 60° bis 90° zu dem zweiten Gehäuse angeordnet sein.

Der Kanal kann bevorzugt mindestens an einer Stelle einen Innendurchmesser von weniger als 3 mm, bevorzugt weniger als 2 mm und weiter bevorzugt weniger als 1 mm aufweisen. Aufgrund eines geringen Innendurchmesser ist der Kanal eingerichtet, durch eine in dem Kanal aufgenommene Flüssigkeit einen Meniskus auszubilden. Ein solcher Meniskus kann eine Flüssigkeit in dem Kanal zurückhalten, solange kein Druckunterschied zwischen dem ersten Innenraum und dem zweiten Innenraum der Vorrichtung herrscht. Hierdurch kann die Abgabe einer Flüssigkeit nach Aufbrechen einer Glasampulle in dem Ampullenbrecher zeitlich besser gesteuert werden. Dies kann den Vorteil haben, ein zu frühes Eindringen von Flüssigkeit in den zweiten Innenraum zu verhindern, um beispielsweise ein unerwünschtes Verkleben des Kanals durch Knochenzement zu verhindern.

In einer Ausführungsform kann die Vorrichtung zwei Ampullenbrecher aufweisen, die jeweils mit einem eigenen Kanal mit der Kartusche verbunden sein können. In einer Ausführungsform sind hierbei die beiden Kanäle in einem Winkel von 180° zueinander angeordnet. Die Vorrichtung kann beispielsweise in Bezug auf die beiden Ampullenbrecher und die beiden Kanäle eine spiegelsymmetrische oder punktsymmetrische Form aufweisen.

Wenn die Vorrichtung einen Verschluss aufweist, kann der Kanal auf der dem Verschluss gegenüberliegenden Seite des zweiten Gehäuses angeordnet sein.

Der Ampullenbrecher weist bevorzugt ein erstes Ende und ein zweites Ende auf, wobei das erste Ende und das zweite Ende bevorzugt gegenüberliegend zueinander angeordnet sind. Das zweite Ende ist bevorzugt eingerichtet, während der Benutzung der Vorrichtung nach unten ausgerichtet zu werden, um die in einer Glasampulle enthaltene Flüssigkeit innerhalb des ersten Gehäuses nach unten in den Kanal abzugeben. "Unten" bezeichnet in diesem Zusammenhang die Richtung der Schwerkraft. "Oben" bezeichnet in entsprechender Weise die entgegengesetzte Richtung zur Schwerkraft. Dementsprechend kann die Vorrichtung bevorzugt eingerichtet sein, während der Benutzung der Vorrichtung mit dem ersten Ende nach oben ausgerichtet zu werden.

Der Ampullenbrecher weist ein erstes Gehäuse auf, in welchem die einzelnen hierin beschriebenen Elemente des Ampullenbrechers angeordnet sein können. Das erste Gehäuse weist einen ersten Innenraum auf, in welchem eine Halterung zur Aufnahme einer Glasampulle angeordnet ist.

Der Ampullenbrecher ist zur Aufnahme einer Glasampulle eingerichtet. Eine Glasampulle ist ein Behälter aus Glas, welcher eine darin enthaltene Flüssigkeit gasdicht und flüssigkeitsdicht nach außen abgrenzen kann. Glasampullen haben üblicherweise einen Grundkörper mit im Wesentlichen zylindrischer Form. Die hierin beschriebenen Glasampullen weisen bevorzugt einen Ampullenkopf auf, der durch einen Ampullenhals mit dem übrigen Glaskörper, d. h. dem Grundkörper, der Glasampulle verbunden ist. Der Ampullenhals weist einen niedrigeren Durchmesser als der Grundkörper auf. Der Ampullenhals kann eine Sollbruchstelle aufweisen, wobei der Ampullenkopf durch Abbruch an dieser Sollbruchstelle von der Glasampulle trennbar ist. Die Sollbruchstelle definiert daher eine Öffnung, aus der die Flüssigkeit nach Abbruch des Ampullenkopfs aus der Glasampulle herausfließen kann. Die Sollbruchstelle ist bevorzugt ringförmig. Der Innendurchmesser der Sollbruchstelle kann bevorzugt 4 bis 7 mm betragen, beispielsweise 5 bis 6 mm. Ein Durchmesser der Sollbruchstelle von mehr als 4 mm kann das Herausfließen der in der Glasampulle befindlichen Flüssigkeit erleichtern. In diesem Fall kann die Flüssigkeit auch bei senkrechter Orientierung der Glasampulle vollständig aus der Glasampulle herausfließen.

Das Herausfließen der Flüssigkeit kann auch durch die Anordnung der Glasampulle in der Halterung verbessert werden. Diese Verbesserung des Herausfließens der Flüssigkeit kann eine höhere Fließgeschwindigkeit oder eine vollständigere Entleerung der Glasampulle umfassen. Bevorzugt kann die Halterung eingerichtet sein, die Glasampulle während der Benutzung der hierin beschriebenen Vorrichtung in einem Winkel von 20° bis 50° zur Senkrechten (d. h. in Richtung der Schwerkraft) zu halten. In einer Ausführungsform ist die Vorrichtung eingerichtet, eine Glasampulle in einem Winkel von 20° bis 50° zur Senkrechten aufzubrechen und zu entleeren.

In einer Ausführungsform ist die Vorrichtung eingerichtet, eine Glasampulle, die in einem Winkel von 20° bis 50° zu einer Längsachse der Kartusche angeordnet ist, aufzubrechen und zu entleeren.

Durch eine wie oben beschriebene gewinkelte Anordnung kann die Flüssigkeit aus der Glasampulle auch in Fällen erreicht werden, in welchen der Innendurchmesser der Sollbruchstelle weniger als 4 mm beträgt, beispielsweise weniger als 3 mm.

Die Verwendung von Glasampullen kann gegenüber Folienbeuteln vorteilhaft sein, da Glasampullen einen Austritt der Monomerflüssigkeit aus der Ampulle durch Diffusion vollständig über einen Zeitraum von Jahren verhindern und kostengünstig hergestellt und befüllt werden können.

Der Ampullenbrecher weist eine Halterung zur Aufnahme einer Glasampulle auf. Die Halterung kann eine dauerhafte Lagerung einer Glasampulle in der Vorrichtung ermöglichen. Die Halterung kann beispielsweise eingerichtet sein, eine handelsübliche Glasampulle kraftschlüssig oder formschlüssig zu fixieren, sodass die Glasampulle unbeweglich in der Halterung positioniert werden kann. In einer Ausführungsform kann eine nach deren Aufnahme in der Halterung nicht mehr, oder nur noch nur noch mithilfe eines dafür vorgesehenen Kolbens innerhalb des ersten Gehäuses bewegt werden. Hierzu kann die Halterung eine Arretierung zu Fixierung einer Glasampulle aufweisen. Beispielsweise kann die Vorrichtung zum Halten einer Glasampulle mit einer elastischen Nase oder Strebe ausgestattet sein, welche zum Beispiel in Richtung des ersten Endes des Ampullenbrechers angeordnet sein kann.

In einer Ausführungsform ist die Halterung eingerichtet, eine Glasampulle in einem Winkel von 20° bis 50° zu einer Längsachse der Kartusche aufzunehmen. In einer Ausführungsform ist die Halterung eingerichtet, eine Glasampulle in einem Winkel von 0° bis 20° zu einer Längsachse der Kartusche aufzunehmen.

Die Vorrichtung kann weiterhin einen Kragen aufweisen, der zum Beispiel in Richtung des zweiten Endes des Ampullenbrechers angeordnet sein kann. Der Kragen kann so dimensioniert sein, dass der Ampullenkopf durch den Kragen geschoben werden kann, wobei der Kragen dem Grundkörper der Ampulle zurückhält. Eine Glasampulle kann beispielsweise zwischen einem solchen Kragen und einer Nase oder Strebe, oder einem vergleichbaren Element zu diesem Zweck, in der Halterung gehalten werden.

Die Vorrichtung kann weiterhin einen Vorsprung aufweisen. Der Vorsprung ist bevorzugt zum Aufbrechen einer in der Halterung aufnehmbaren Glasampulle ausgebildet und eingerichtet. Der Vorsprung kann hierzu derart in dem ersten Gehäuse angeordnet sein, dass er die freie Bewegung einer in der Halterung aufnehmbaren Glasampulle einschränkt. Insbesondere kann der Vorsprung so angeordnet sein, dass der Kopf einer in der Halterung aufnehmbaren Glasampulle mit dem Vorsprung kollidiert, wenn die Glasampulle innerhalb der Vorrichtung in Richtung des zweiten Endes des Ampullenbrechers bewegt wird. Der Vorsprung kann sich beispielsweise vom Rand des ersten Gehäuses in Richtung der Mitte des Innenraums des ersten Gehäuses erstrecken.

Die Vorrichtung kann weiterhin einen Auslass aufweisen, um Flüssigkeit aus dem Kanal des ersten Gehäuses in das zweite Gehäuse abzugeben. Der Auslass kann eine Öffnung, eine Düse, eine Röhre oder eine Schlauchleitung aufweisen. Der Auslass kann alternativ oder zusätzlich auch einen Adapter zum Anschluss an eine Kartusche mit Knochenzementpulver aufweisen.

Der Ampullenbrecher kann einen in dem ersten Gehäuse beweglich angeordneten Kolben aufweisen. Dieser Kolben ist bevorzugt ausgebildet und eingerichtet, eine in der Halterung aufnehmbare Glasampulle durch Druck der Glasampulle gegen einen Vorsprung aufzubrechen. Dabei kann der Kolben die Halterung in Richtung des Vorsprung bewegen, um den Kopf der Glasampulle vom Grundkörper der Glasampulle abzubrechen. Weiterhin ist der Kolben bevorzugt ausgebildet und eingerichtet, eine Flüssigkeit aus der wie oben beschrieben aufgebrochenen Glasampulle aus dem Auslass des Kanals des ersten Gehäuses zu pumpen. Demnach kann der Kolben zwei Funktionen ausüben, nämlich das Aufbrechen einer in der Vorrichtung aufnehmbaren Glasampulle, und die Abgabe der in einer solchen Glasampulle enthaltenen Flüssigkeit aus dem Ampullenbrecher an die Kartusche.

Der Ampullenbrecher weist bevorzugt weiterhin einen Griff auf, welcher einem Benutzer das manuelle Greifen und Bewegen des Kolbens ermöglicht. Der Griff kann beispielsweise am ersten Ende des Ampullenbrechers, bevorzugt am gegenüberliegenden Ende zum Auslass des Ampullenbrechers, angeordnet sein.

In einer bevorzugten Ausführungsform weist die Vorrichtung ein Ventil auf. Das Ventil kann eingerichtet sein, Luft von außen in das erste Gehäuse oder das zweite Gehäuse einströmen zu lassen. Weiterhin kann das Ventil eingerichtet sein, durch Druck eines Kolbens auf die in das Gehäuse eingeströmte Luft eine Flüssigkeit aus einer in der Vorrichtung aufgebrochenen Glasampulle aus dem ersten Gehäuse zu pumpen. Das Ventil kann einen Filter aufweisen, um zu verhindern, dass Staub oder Keime von außen in die Vorrichtung gelangen. Das Ventil ist bevorzugt am Ampullenbrecher angeordnet, weiter bevorzugt am ersten Ende des Ampullenbrechers.

Das Ventil kann bevorzugt ein Einwegeventil sein, d. h. ein Ventil, welches in eine Richtung luftdurchlässig und in eine andere Richtung luftundurchlässig ist. Das Ventil kann beispielsweise ein Kugelventil, Lippenventil oder Plattenventil sein.

Die Vorrichtung kann weiterhin einen Auffangbereich aufweisen. Der Auffangbereich kann in dem ersten Gehäuse angeordnet und zur Aufnahme eines durch Wirkung eines Aufbrechelements abgebrochenen Teils einer Glasampulle eingerichtet sein. Der Auffangbereich ist bevorzugt ausreichend groß dimensioniert, sodass der Ampullenkopf einer in der Vorrichtung aufnehmbaren Glasampulle sich um etwa 90° seitlich drehen kann, und die darin enthaltene Flüssigkeit aus dem Ampullenkopf austreten kann, ohne den Ampullenkopf selbst vollständig zu zerbrechen. Beispiele für einen solchen Auffangbereich sind in EP2404864B1 beschrieben. Bevorzugt sind Höhe und Querschnitt des Auffangbereichs mindestens so groß wie die Höhe des Ampullenkopfs bis zur Sollbruchstelle, sodass der Auffangbereich geeignet ist, eine Drehung des abgebrochenen Ampullenkopfs im Auffangbereich zu ermöglichen, wie es in EP2404864B1 ausführlicher beschrieben ist.

In einer Ausführungsform weist die Vorrichtung weiterhin ein Sieb auf, welches angrenzend an den Auffangbereich angeordnet ist. Das Sieb ist bevorzugt eingerichtet, Bruchstücke aus einer in der Vorrichtung aufgebrochenen Glasampulle, beispielsweise einen Ampullenkopf, in der Vorrichtung zurückzuhalten. Hierdurch kann beispielsweise verhindert werden, dass mithilfe der Vorrichtung hergestellter Knochenzement durch Glassplitter verunreinigt wird. Das Sieb kann beispielsweise ein Metalldrahtgeflecht oder ein Netz aus einem Kunststoffgewebe sein. Das Sieb kann auch eine mit zweckmäßig dimensionierten Löchern versehene Scheibe aus Metall oder Kunststoff sein. Weiterhin können offenporige Schaumstoffe wie zum Beispiel Porex verwendet werden. Bevorzugt sollte das Sieb aus einem Material gefertigt sein, welches durch die in einer aufnehmbaren Glasampulle befindliche Flüssigkeit nicht angegriffen wird.

In einer weiteren Ausführungsform weist die Vorrichtung weiterhin einen Sammelbereich auf, um Flüssigkeit aus einer in der Vorrichtung aufgebrochenen Glasampulle aufzunehmen. Der Sammelbereich ist bevorzugt fluidleitend mit der Halterung verbunden, sodass Flüssigkeit aus einer in der Halterung aufnehmbaren Glasampulle in den Sammelbereich fließen kann. Dabei kann die Flüssigkeit bevorzugt aus der Richtung des ersten Endes der Vorrichtung in Richtung des zweiten Endes der Vorrichtung fließen. Der Sammelbereich kann dazu angeordnet und eingerichtet sein, eine Flüssigkeit aus einer in der Vorrichtung aufgebrochenen Glasampulle aufzunehmen und an den Auslass der Vorrichtung zu leiten. Der Sammelbereich kann durch ein wie oben dargestelltes Sieb vom Auffangbereich abgegrenzt sein, wobei das Sieb eine fluidleitende Verbindung zwischen dem Auffangbereich und dem Sammelbereich bilden kann.

In einer Ausführungsform ist die Vorrichtung zum Mischen von Knochenzement ausgebildet und eingerichtet. Bevorzugt weist die Vorrichtung hierzu weiterhin eine Pulverkomponente zur Zubereitung eines Knochenzements auf. Diese Pulverkomponente kann in einer Kartusche angeordnet sein, wobei die Kartusche über den Kanal mit Flüssigkeit aus einer in dem Ampullenbrecher aufgebrochenen Glasampulle befüllbar sein kann. Ein Auslass des Kanals kann mithilfe eines Adapters mit der Kartusche verbindbar sein, zum Beispiel durch eine Schnapp- oder Schraubverbindung. Zum Mischen der Flüssigkeit und der Pulverkomponente kann weiterhin ein Mischelement, zum Beispiel ein Mischstab, vorgesehen sein, welches die Durchmischung der Flüssigkeit und der Pulverkomponente ermöglicht. Der Mischstab kann einen Handgriff, einen Stab und eine Mischscheibe aufweisen.

In einer Ausführungsform weist der Ampullenbrecher ein erstes Ende und ein zweites Ende auf, wobei das erste Ende dem zweiten Ende gegenüberliegend angeordnet ist. Bevorzugt ist ein Kolben an dem ersten Ende angeordnet. Dies betrifft bevorzugt einen Teil des Kolbens, der einen Griffbereich zum Anfassen und manuellen Bewegen des Kolbens bildet. Dieser Griffbereich wird hierin auch als "Griff" bezeichnet. Bevorzugt ist ein Kanal an dem zweiten Ende des Ampullenbrechers angeordnet.

In einer Ausführungsform weist die Vorrichtung einen Kolben auf, und ist ausgebildet und eingerichtet, mit einem einzigen Hub des Kolbens den gesamten Inhalt einer in der Halterung aufnehmbaren oder aufgenommenen Glasampulle aus dem ersten Gehäuse in das zweite Gehäuse zu pumpen. Dies kann insbesondere dadurch erreicht werden, dass das Hubvolumen des Kolbens ausreichend groß im Verhältnis zum Volumen einer abzugebenden Flüssigkeit in der Glasampulle dimensioniert ist, beispielsweise mindestens gleich groß.

In einer Ausführungsform ist eine Glasampulle kraftschlüssig, formschlüssig oder stoffschlüssig in der Halterung befestigt oder fixierbar. Eine Glasampulle kann beispielsweise durch Kleben oder Klemmen in der Vorrichtung fixierbar sein oder befestigt sein. Wie oben dargestellt kann eine Glasampulle beispielsweise im Bereich des Ampullenhalses von einem Kragen gehalten sein, und gleichzeitig am gegenüberliegenden Boden der Ampulle von einer Arretierung, zum Beispiel einer Strebe, einem Vorsprung oder dergleichen, unter Spannung gehalten werden.

In einer Ausführungsform ist die der Ampullenbrecher zum gleichzeitigen Aufbrechen und Abgeben von Flüssigkeit aus zwei Glasampullen ausgebildet und eingerichtet. In einer Ausführungsform ist die Vorrichtung eingerichtet, mithilfe eines einzelnen Kolbens oder mithilfe zweier verbundener Kolben gleichzeitig zwei Glasampullen aufzubrechen und die darin enthaltene Flüssigkeit aus dem Ampullenbrecher in die Kartusche abzugeben.

Die Vorrichtung kann weiterhin ein Element zum Aufbau eines Druckunterschieds zwischen dem ersten Innenraum und dem zweiten Innenraum aufweisen, um eine Flüssigkeit durch den Kanal zu überführen. Dieses Element kann beispielsweise in Form eines hierin beschriebenen Kolbens ausgestaltet sein. Die Vorrichtung kann auch einen Anschluss zur Verbindung mit einer Pumpe aufweisen. Die Vorrichtung kann einen Vakuumanschluss zur Verbindung mit einer im medizinischen Umfeld üblichen Vakuumquelle aufweisen. Die Vorrichtung kann eine mechanische oder elektrische Pumpe aufweisen. Die Vorrichtung kann ein flexibles Element aufweisen, welches eingerichtet sein kann, durch Zusammendrücken des flexiblen Elements einen Überdruck in dem ersten Innenraum zu erzeugen. Dies kann beispielsweise nach Art eines Peleusballs erfolgen, der üblicherweise in Laboren als Pipettierhilfe eingesetzt wird. Das Element zum Aufbau eines Druckunterschieds zwischen dem ersten Innenraum und dem zweiten Innenraum kann in dem Ampullenbrecher oder in der Kartusche angeordnet sein. Das Element kann operativ mit dem ersten Innenraum oder dem zweiten Innenraum verbunden sein. Ein Beispiel eines solchen Elements zum Aufbau eines Druckunterschieds ist in WO2010/089621A1 offenbart.

In einer Ausführungsform weist die Kartusche einen Anschluss für eine Vakuumquelle auf. In einer Ausführungsform weist der Ampullenbrecher einen Anschluss für ein druckbeaufschlagtes Medium auf.

In einer Ausführungsform enthält die Vorrichtung eine Glasampulle. Die Glasampulle kann dabei in der Halterung aufgenommen sein, wie vorangehend erläutert. Die Glasampulle kann eine Flüssigkeit enthalten, beispielsweise eine Monomerlösung zur Herstellung eines Knochenzements, bevorzugt eines PMMA-Knochenzements.

Wenn die Vorrichtung sowohl eine Glasampulle mit einer Flüssigkeit als auch eine zweite Komponente zur Herstellung eines Gemischs daraus, zum Beispiel eine Flüssigkeit und ein Pulver zur Herstellung von Knochenzement enthält, oder allgemein alle Ausgangsstoffe für den beabsichtigen Zweck der Vorrichtung enthält, kann die Vorrichtung auch als "Full-Prepacked-Mischsystem" bezeichnet werden. Sie ist demnach unmittelbar einsatzbereit zur Herstellung von Knochenzement, ohne weitere Ausgangsstoffe hinzufügen zu müssen. Eine solche Vorrichtung dient bevorzugt zum Lagern der enthaltenen Komponenten sowie zum Mischen und Austragen von Knochenzement, insbesondere Polymethylmethacrylat-Knochenzement. Es sind aber auch andere Anwendungsbereiche im medizinischen oder nichtmedizinischen Gebiet denkbar, in denen eine Flüssigkeit aus einer Glasampulle abgegeben werden soll. Das Pulver kann bevorzugt in der Kartusche angeordnet sein, weiter bevorzugt in dem zweiten Gehäuse, insbesondere in dem zweiten Innenraum.

Die Vorrichtung weist weiterhin eine Kartusche auf. Die Kartusche ist ein geschlossener Behälter, der bevorzugt zur Zubereitung, insbesondere zur Anmischung, von Knochenzement aus einem Pulver und einer Flüssigkeit eingerichtet ist. In einer Ausführungsform weist die Vorrichtung ein Verbindungselement auf, um den Ampullenbrecher mit der Kartusche zu verbinden. Das Verbindungselement kann eine Klemme aufweisen, um das erste Gehäuse des Ampullenbrechers mit dem zweiten Gehäuse der Kartusche zu verbinden. Die Vorrichtung kann auch einen Adapter zum Anschluss des Auslass des Ampullenbrechers an die Kartusche aufweisen. Die Kartusche kann einen Mischstab zur Vermischung eines Pulvers mit einer Flüssigkeit enthalten, der eine manuelle oder maschinelle Vermischung der Flüssigkeit und der Pulverkomponente ermöglicht. Der Mischstab kann beispielsweise einen Griff aufweisen, der an einer Außenseite der Vorrichtung angeordnet sein kann. Der Mischstab kann weiterhin einen Stab umfassen, der den Griff mit einer innerhalb des zweiten Gehäuses angeordneten Scheibe verbindet. Die Scheibe kann von einem Benutzer des Mischsystems mithilfe des Griffs innerhalb des zweiten Innenraums der Kartusche bewegt werden, um hierdurch die Flüssigkeit mit der Pulverkomponente zu mischen. Der Mischstab kann mithilfe einer Verschraubung an der Kartusche abgebracht sein.

Die Kartusche weist ein Gehäuse auf, welches hierin als "zweites Gehäuse" bezeichnet ist, um es von dem Gehäuse des Ampullenbrechers, welches hierin als "erstes Gehäuse" bezeichnet ist, zu unterscheiden. Das zweite Gehäuse weist einen zweiten Innenraum auf. Das zweite Gehäuse kann gegebenenfalls mehrteilig ausgebildet sein, wie in den Figuren näher verdeutlicht ist. Das zweite Gehäuse kann bevorzugt die Form eines Hohlzylinders aufweisen.

Ein weiterer nicht beanspruchter Aspekt betrifft ein Verfahren zur Abgabe einer Flüssigkeit aus einer Glasampulle mit Hilfe einer oben beschriebenen Vorrichtung, wobei das Verfahren die folgenden Schritte aufweist,
- Fixieren der Glasampulle in einer Halterung der Vorrichtung;
- Aufbrechen der Glasampulle mithilfe des Aufbrechelements;
- Abgabe der Flüssigkeit aus der aufgebrochenen Glasampulle durch einen Kanal aus einem ersten Innenraum in einem ersten Gehäuse in einen zweiten Innenraum eines zweiten Gehäuses der Vorrichtung,
wobei der fluidleitende Kanal angeordnet und eingerichtet ist, die Flüssigkeit in einem Sammelbereich zurückzuhalten, und die Flüssigkeit an den zweiten Innenraum abzugeben, sobald zwischen dem Sammelbereich und dem zweiten Innenraum ein Druckunterschied anliegt.

In einer Ausführungsform umfasst das Verfahren einen weiteren Schritt, in welchem die Flüssigkeit in einem zweiten Gehäuse der Vorrichtung mit einer Pulverkomponente gemischt wird. Das Verfahren kann beispielsweise zur Zubereitung eines Knochenzements verwendet werden.

Die Flüssigkeit ist bevorzugt eine Monomerflüssigkeit zur Zubereitung eines Knochenzements, beispielsweise eines PMMA-Knochenzements. Das Verfahren ist bevorzugt ein Verfahren zur Herstellung von Knochenzement. Das Verfahren wird bevorzugt vollständig *in vitro,* d.h. außerhalb des menschlichen Körpers, durchgeführt.

Das Aufbrechen der Glasampulle kann mithilfe eines Kolbens erfolgen, der die Glasampulle gegen einen Vorsprung drückt und dadurch den Ampullenkopf abbricht. Das Aufbrechen der Glasampulle kann auch mithilfe einer Taste erfolgen, die in das erste Gehäuse eingedrückt wird, und dadurch den Ampullenkopf abbricht. Das Aufbrechen der Glasampulle kann auch mithilfe eines biegsamen Teils des ersten Gehäuses erfolgen, wobei durch das Biegen des ersten Gehäuses der Ampullenkopf abbricht. Auch weitere im Fachgebiet bekannte Verfahren zum Aufbrechen einer Glasampulle können verwendet werden.

Die erfindungsgemäße Vorrichtung ist bevorzugt ein Mischsystem für Knochenzement, welches einen hierin beschriebenen Ampullenbrecher und eine hierin beschriebene Kartusche aufweist. Ein solches Mischsystem kann zwei Komponenten zur Herstellung von Knochenzement enthalten, zum Beispiel eine Monomerflüssigkeit und eine Pulverkomponente zur Herstellung von PMMA-Knochenzement.

Ein beispielhaftes Mischsystem enthält beispielsweise einen hierin beschriebenen Ampullenbrecher, sowie weiterhin eine Mischeinheit, aufweisend eine hohlzylinderförmige Kartusche mit einem Innenraum, wobei in dem Innenraum ein Knochenzementpulver angeordnet ist, und einen Kartuschenkopf, welcher den Innenraum an einem proximalem Kartuschenende fluidleitend verschließt, und ein Leitungsmittel, welches den Innenraum der Mischeinheit fluidleitend mit dem Auslass der Vorrichtung verbindet, wobei sich das Leitungsmittel bevorzugt durch eine Kartuschenkopfdurchführung des Kartuschenkopfs der Mischeinheit erstreckt und das Leitungsmittel und die Kartuschenkopfdurchführung einen Formschluss ausbilden.

Das Mischsystem enthält beispielsweise in einer Ausführungsform
- einen Ampullenbrecher mit einem ersten Gehäuse, wobei das erste Gehäuse einen ersten Innenraum aufweist,
- eine in dem ersten Gehäuse angeordnete Halterung zur Aufnahme einer Glasampulle,
- ein in dem ersten Gehäuse angeordnetes Aufbrechelement, das zum Aufbrechen einer in der Halterung aufnehmbaren Glasampulle ausgebildet und eingerichtet ist,
- ein in dem ersten Gehäuse angeordneter Sammelbereich, der zur Aufnahme einer Flüssigkeit aus einer aufgebrochenen Glasampulle ausgebildet und eingerichtet ist,
- eine Kartusche, die zur Aufnahme einer Pulverkomponente eines Knochenzements eingerichtet ist, wobei die Kartusche ein zweites Gehäuse mit einem zweiten Innenraum aufweist,
- einen fluidleitenden Kanal zur Abgabe einer Flüssigkeit aus dem Sammelbereich in den zweiten Innenraum, wobei der fluidleitende Kanal angeordnet und eingerichtet ist, die Flüssigkeit in dem Sammelbereich zurückzuhalten, und die Flüssigkeit an den zweiten Innenraum abzugeben, sobald zwischen dem Sammelbereich und dem zweiten Innenraum ein Druckunterschied anliegt,
- und ein Leitungsmittel, welches den ersten Innenraum fluidleitend mit dem zweiten Innenraum verbindet, wobei sich das Leitungsmittel bevorzugt durch eine Durchführung der Kartusche erstreckt und das Leitungsmittel und die Durchführung einen Formschluss ausbilden.

Die Durchführung kann an einem Kartuschenkopf der Kartusche angeordnet sein.

Gemäß dieser Ausführungsform kann mithilfe des Aufbrechelements eine Glasampulle aufgebrochen werden, und die darin enthaltene Flüssigkeit mithilfe eines Leitungsmittels in den zweiten Innenraum transferiert werden.

Das Leitungsmittel kann beispielsweise eine Schlauchverbindung sein, welche innerhalb des Mischsystems einzelne Elemente des Mischsystems fluidleitend miteinander verbindet. Das Leitungsmittel kann bevorzugt ein elastisches Polymer aufweisen, oder daraus bestehen. Das elastische Polymer kann beispielsweise ein Silikon, PE, PP, PU, PA oder FEP umfassen.

In einer weiteren Ausführungsform enthält das Mischsystem
- einen Ampullenbrecher mit einem ersten Gehäuse, wobei das erste Gehäuse einen ersten Innenraum aufweist,
- eine in dem ersten Gehäuse angeordnete Halterung zur Aufnahme einer Glasampulle,
- ein in dem ersten Gehäuse angeordnetes Aufbrechelement, das zum Aufbrechen einer in der Halterung aufnehmbaren Glasampulle ausgebildet und eingerichtet ist,
- ein in dem ersten Gehäuse angeordneter Sammelbereich, der zur Aufnahme einer Flüssigkeit aus einer aufgebrochenen Glasampulle ausgebildet und eingerichtet ist,
- eine Kartusche, die zur Aufnahme einer Pulverkomponente eines Knochenzements eingerichtet ist, wobei die Kartusche ein zweites Gehäuse mit einem zweiten Innenraum aufweist,
- einen fluidleitenden Kanal zur Abgabe einer Flüssigkeit aus dem Sammelbereich in den zweiten Innenraum, wobei der fluidleitende Kanal angeordnet und eingerichtet ist, die Flüssigkeit in dem Sammelbereich zurückzuhalten, und die Flüssigkeit an den zweiten Innenraum abzugeben, sobald zwischen dem Sammelbereich und dem zweiten Innenraum ein Druckunterschied anliegt,
- einen Kartuschenkopf, welcher den zweiten Innenraum an einem proximalem Kartuschenende fluidleitend verschließt,
- und einen hohlzylinderförmigen Adapter, der zur reversiblen Verbindung eines proximalen Kartuschenendes mit einem Auslass des Kanals eingerichtet ist.

Gemäß dieser Ausführungsform kann mithilfe des Aufbrechelements eine Glasampulle aufgebrochen werden, und die darin enthaltene Flüssigkeit mithilfe eines reversibel verbindbaren Adapters in den zweiten Innenraum transferiert werden.

In einer weiteren Ausführungsform enthält das Mischsystem
- einen Ampullenbrecher mit einem ersten Gehäuse, wobei das erste Gehäuse einen ersten Innenraum aufweist,
- eine in dem ersten Gehäuse angeordnete Halterung zur Aufnahme einer Glasampulle,
- ein in dem ersten Gehäuse angeordnetes Aufbrechelement, das zum Aufbrechen einer in der Halterung aufnehmbaren Glasampulle ausgebildet und eingerichtet ist,
- ein in dem ersten Gehäuse angeordneter Sammelbereich, der zur Aufnahme einer Flüssigkeit aus einer aufgebrochenen Glasampulle ausgebildet und eingerichtet ist,
- eine Kartusche, die zur Aufnahme einer Pulverkomponente eines Knochenzements eingerichtet ist, wobei die Kartusche ein zweites Gehäuse mit einem zweiten Innenraum aufweist,
- einen fluidleitenden Kanal zur Abgabe einer Flüssigkeit aus dem Sammelbereich in den zweiten Innenraum, wobei der fluidleitende Kanal angeordnet und eingerichtet ist, die Flüssigkeit in dem Sammelbereich zurückzuhalten, und die Flüssigkeit an den zweiten Innenraum abzugeben, sobald zwischen dem Sammelbereich und dem zweiten Innenraum ein Druckunterschied anliegt,
- einen Kartuschenkopf, welcher den zweiten Innenraum an einem proximalem Kartuschenende fluidleitend verschließt,
- ein Fußteil, welches als Aufstandsfläche für das Mischsystem eingerichtet ist,
- einen Kartuschenkopf, der mit dem Fußteil verbindbar ist oder verbunden ist,
- eine Einspritzdüse, die im Fußteil angeordnet ist und sich in den Kartuschenkopf erstreckt,
- und ein Leitungsmittel, das mit der Einspritzdüse fluidleitend verbunden ist.

Gemäß dieser Ausführungsform kann mithilfe des Aufbrechelements eine Glasampulle aufgebrochen werden, und die darin enthaltene Flüssigkeit mithilfe eines Leitungsmittels über eine Einspritzdüse in den zweiten Innenraum transferiert werden.

Das Mischsystem ist bevorzugt ein "Full-Prepacked-Mischsystem" wie oben beschrieben. Ein solches Mischsystem kann in einer sterilen und hermetisch verschlossenen Verpackung bereitgestellt werden. Auch das Mischsystem selbst und die enthaltenen KnochenzementKomponenten können dabei steril bereitgestellt werden.

In einer Ausführungsform kann durch die Bewegung eines Kolbens eine in der Halterung aufgenommene Glasampulle durch Druck gegen einen Vorsprung aufgebrochen werden, um eine darin enthaltene Flüssigkeit, beispielsweise eine Monomerflüssigkeit zur Zubereitung von Knochenzement, aus der Glasampulle freizugeben. Weiterhin kann durch die Bewegung des Kolbens diese Flüssigkeit durch den Kanal und gegebenenfalls durch ein oben dargestelltes Leitungsmittel, einen Adapter und/oder eine Einspritzdüse in die Mischeinheit gepumpt werden, und sich dort mit dem dort angeordneten Knochenzementpulver mischen, um aus der Flüssigkeit und der Pulverkomponente einen Knochenzement zu bilden.

Zur Zubereitung eines Knochenzements können die Flüssigkeit aus einer in dem Ampullenbrecher aufgebrochenen Glasampulle und die Pulverkomponente weiterhin in der Kartusche miteinander vermengt werden. Das Mischsystem kann hierzu einen Mischstab enthalten, der eine manuelle oder maschinelle Vermischung der Flüssigkeit und der Pulverkomponente ermöglicht. Der Mischstab kann beispielsweise einen Griff aufweisen, der an der Außenseite des Mischsystems angeordnet sein kann. Der Mischstab kann weiterhin einen Stab umfassen, der den Griff mit einer innerhalb des Mischsystems angeordneten Scheibe verbindet. Die Scheibe kann von einem Benutzer des Mischsystems mithilfe des Griffs innerhalb des Innenraums der hohlzylinderförmigen Kartusche bewegt werden, um hierdurch die Flüssigkeit mit der Pulverkomponente zu mischen.

Das Mischsystem kann weiterhin ein Sieb aufweisen, um Bruchstücke der aufgebrochenen Ampulle zurückzuhalten, wie zuvor ausführlicher beschrieben.

Das Mischsystem kann weiterhin einen Auffangbereich aufweisen, wie zuvor beschrieben. Der Auffangbereich kann in dem Gehäuse angeordnet und zur Aufnahme eines durch Wirkung des Kolbens abgebrochenen Teils einer Glasampulle eingerichtet sein. Der Auffangbereich ist bevorzugt ausreichend groß dimensioniert, sodass der Ampullenkopf einer in der Vorrichtung aufgebrochenen Glasampulle sich um etwa 90° seitlich drehen kann, und die darin enthaltene Flüssigkeit aus dem Ampullenkopf austreten kann, ohne den Ampullenkopf selbst vollständig zu zerbrechen.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

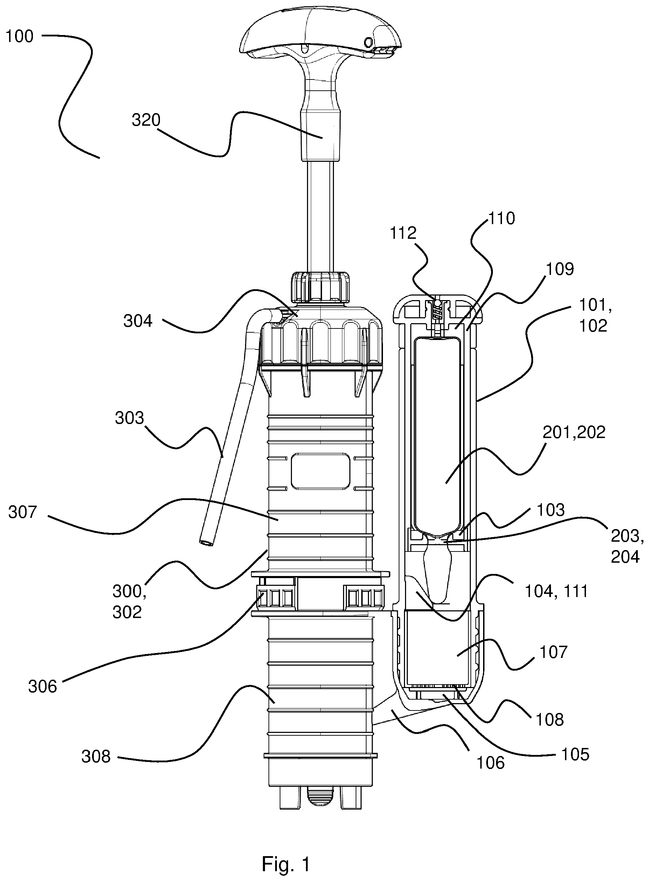

**Figur 1** zeigt beispielhaft eine erste Ausführungsform einer erfindungsgemäßen Vorrichtung **100** in einer seitlichen Ansicht, wobei der Ampullenbrecher als Querschnitt dargestellt ist. Die Vorrichtung weist einen Ampullenbrecher **101** mit einem ersten Gehäuse **102** auf. Das erste Gehäuse **102** weist einen ersten Innenraum **110** auf, in dem eine Halterung **103** zur Aufnahme einer Glasampulle **201** angeordnet ist. Die Glasampulle **201** weist einen Ampullenhals **203** mit einer Sollbruchstelle **204** auf. Die Glasampulle **201** enthält eine Flüssigkeit **202.** Die Halterung **103** weist im hier dargestellten Beispiel einen Kragen auf, welcher die Glasampulle **201** im Bereich des Ampullenhalses **203** umgibt. Der Ampullenbrecher **101** weist ein Aufbrechelement **104** auf, welches im hier gezeigten Beispiel als Vorsprung **111** ausgestaltet ist. Der Ampullenbrecher **101** weist hier einen Kolben **109** auf, der eingerichtet ist, die Glasampulle **201** gegen den Vorsprung **111** zu schieben, um die Glasampulle **201** aufzubrechen. Der Ampullenbrecher weist im hier gezeigten Beispiel weiterhin ein Ventil **112** auf, welches Luft von außen in den ersten Innenraum **110** leiten kann, aber keine Luft aus dem ersten Innenraum **110** nach außen gelangen lässt. Somit kann der Kolben **109** sowohl zum Aufbrechen einer Glasampulle **201** und zum Pumpen der darin enthaltenen Flüssigkeit **202** aus dem Ampullenbrecher **101** dienen. Der Ampullenbrecher **101** weist einen Auffangbereich **107** zur Aufnahme eines abgebrochenen Ampullenkopfs auf. Der Auffangbereich **107** ist nach unten durch ein Sieb **108** begrenzt, um den Ampullenkopf oder andere Glasbruchstücke einer aufgebrochenen Glasampulle im Ampullenbrecher **101** zurückzuhalten. Das Sieb **108** ist durchlässig für die Flüssigkeit **202.** Auf der anderen Seite des Siebs **108** befindet sich ein Sammelbereich **105** zur Aufnahme der Flüssigkeit **202.** Somit verbindet das Sieb **108** den Auffangbereich **107** fluidleitend mit dem Sammelbereich **105.** Der Sammelbereich **105** ist weiterhin fluidleitend mit einem Kanal **106** verbunden. Der Kanal **106** verbindet den Sammelbereich **105** fluidleitend mit einem zweiten Innenraum **310** eines zweiten Gehäuses **302** einer Kartusche **300.** Die Kartusche **300** weist ein zweites Gehäuse **302** auf, welches im hier gezeigten Beispiel mehrteilig ausgebildet ist. Das zweite Gehäuse **302** weist daher hier einen ersten Teil **307** und einen zweiten Teil **308** auf, welche durch ein Verbindungselement **306** miteinander verbunden sind, beispielsweise über ein Gewinde. Die Kartusche **300** ist zur Aufnahme eines Pulvers ausgebildet und eingerichtet, beispielsweise einer Pulverkomponente eines PMMA-Knochenzements. Die Pulverkomponente kann in dem zweiten Innenraum **310** angeordnet sein. Die Kartusche **300** weist einen Vakuumanschluss **303** in Form eines Schlauchverbinders auf, der den Anschluss an eine Vakuumpumpe ermöglicht. Auf diese Weise kann ein Druckunterschied zwischen dem ersten Innenraum **110** und dem zweiten Innenraum **310** angelegt werden, um die Flüssigkeit **202** durch den Kanal **106** zu überführen. Der Vakuumanschluss **303** ist an einem Verschluss **304** angeordnet. Der Verschluss **304** verschließt das zweite Gehäuse **302** der Kartusche **300.**

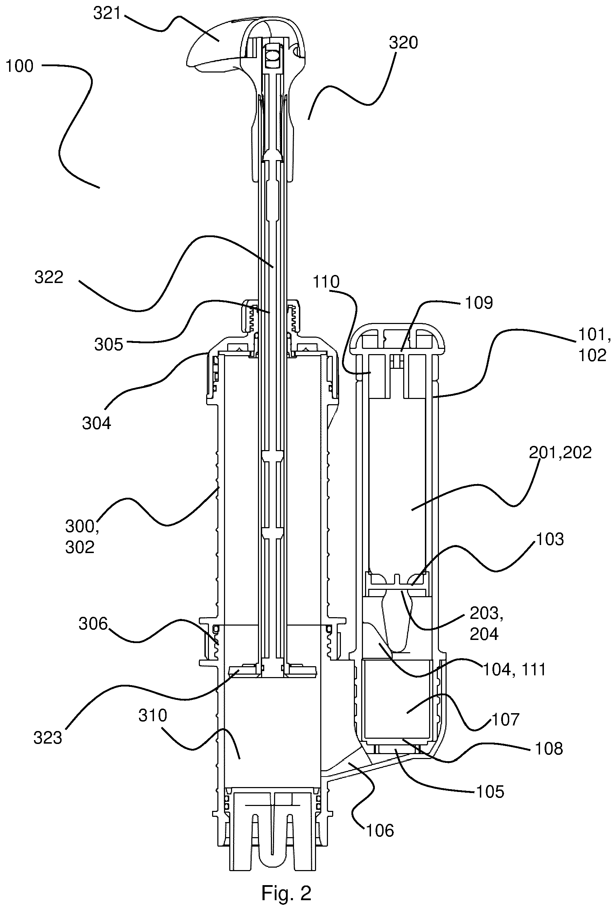

**Figur 2** zeigt eine Ausführungsform einer erfindungsgemäßen Vorrichtung **100** in einer seitlichen Querschnittsansicht. Die Vorrichtung **100** weist einen Ampullenbrecher **101** auf, der über einen Kanal **106** mit einer Kartusche **300** fluidleitend verbunden ist. Die Kartusche **300** weist ein Gehäuse **302** mit einem Innenraum **310** auf. Das Gehäuse **302** weist einen ersten Teil **307** und einen zweiten Teil **308** auf, die über ein Verbindungselement **306** miteinander verbunden sind, wobei das Verbindungselement **306** hier ein Gewinde zur Verbindung des ersten Teils **307** und des zweiten Teils **308** aufweist. Der Innenraum **310** wird durch einen Verschluss **304** begrenzt. Der Verschluss **304** ist auf das Gehäuse **302** geschraubt und somit lösbar mit dem Gehäuse **302** verbunden. Der Verschluss **304** weist weiterhin eine Durchführung **305** zur Aufnahme eines Mischers **320** auf. Der Mischer **320** weist einen Griff **321,** einen Stab **322** und eine Scheibe **323** auf, wobei der Griff **321** außerhalb des zweiten Gehäuses **302** und die Scheibe **323** innerhalb des zweiten Gehäuses **302** angeordnet ist. Der Mischer **320** ermöglicht das Mischen der Flüssigkeit **202** mit einer Pulverkomponente innerhalb des Gehäuses **302.**

Der Ampullenbrecher **101** weist einen Kolben **109** auf, der eine in einer Halterung **103** aufgenommene Glasampulle **201** gegen einen Vorsprung **111** drücken kann, welcher hier als Aufbrechelement **104** dient. Der Verfahrweg des Kolbens **109** ist durch den Vorsprung **111** begrenzt. Der Kolben **109** dient somit zum Aufbrechen der Glasampulle **201.** Darüber hinaus kann mithilfe des Kolbens **109** ein Druckunterschied zwischen dem ersten Innenraum **110** und dem zweiten Innenraum **310** erzeugt werden, um eine Flüssigkeit **202** durch einen Kanal **106** in die Kartusche **300** zu überführen.

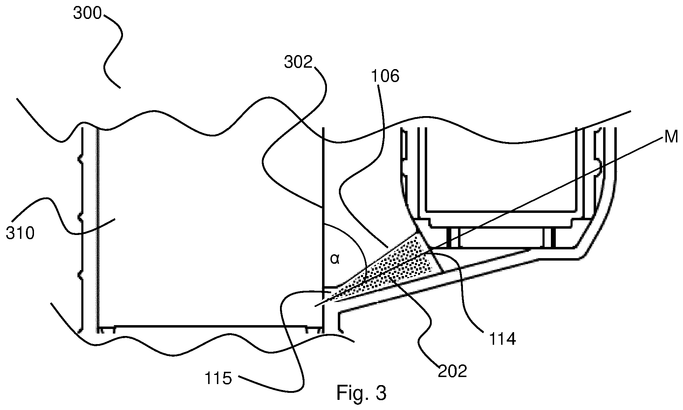

**Figur 3** zeigt einen Ausschnitt einer erfindungsgemäßen Vorrichtung in einer Querschnittsansicht. Der Kanal **106** weist einen Einlass **114** auf, um eine Flüssigkeit aus dem Ampullenbrecher aufzunehmen. Weiterhin weist der Kanal **106** einen Auslass **115** auf, um eine Flüssigkeit an die Kartusche **300** abzugeben. Aufgrund des geringen Innendurchmesser des Kanals **106** bildet sich ein Meniskus in der Flüssigkeit **202** aus, der die Flüssigkeit **202** in dem Kanal **106** hält. Die Ausbildung des Meniskus ist abhängig von der verwendeten Flüssigkeit, der Geometrie des Kanals und seinem Neigungswinkel. Durch den Kanal **106,** genauer gesagt durch die Mittelachse **M** des Kanals **106,** und die angrenzende Außenwand des zweiten Gehäuses **302** wird ein Winkel **α** definiert. Dieser Winkel liegt in einem Bereich von 60° bis 90°. Bei der Verwendung üblicher Monomerlösungen für PMMA-Knochenzemente bildet sich in diesem Winkelbereich ein Meniskus in dem Kanal aus, insbesondere wenn der Kanal einen Innendurchmesser von 3 mm oder weniger aufweist. Erst durch Beauftragung der Vorrichtung mit einem Druckunterschied zwischen dem ersten Innenraum **110** und dem zweiten Innenraum **310** wird die Flüssigkeit **202** vollständig durch den Kanal **106** in die Kartusche **300** überführt. Hierdurch kann der Abgabezeitpunkt der Flüssigkeit **202** in die Kartusche **300,** und somit der Zeitpunkt des Kontakts der Flüssigkeit mit einer Pulverkomponente in der Kartusche **300,** durch den Anwender gesteuert werden.

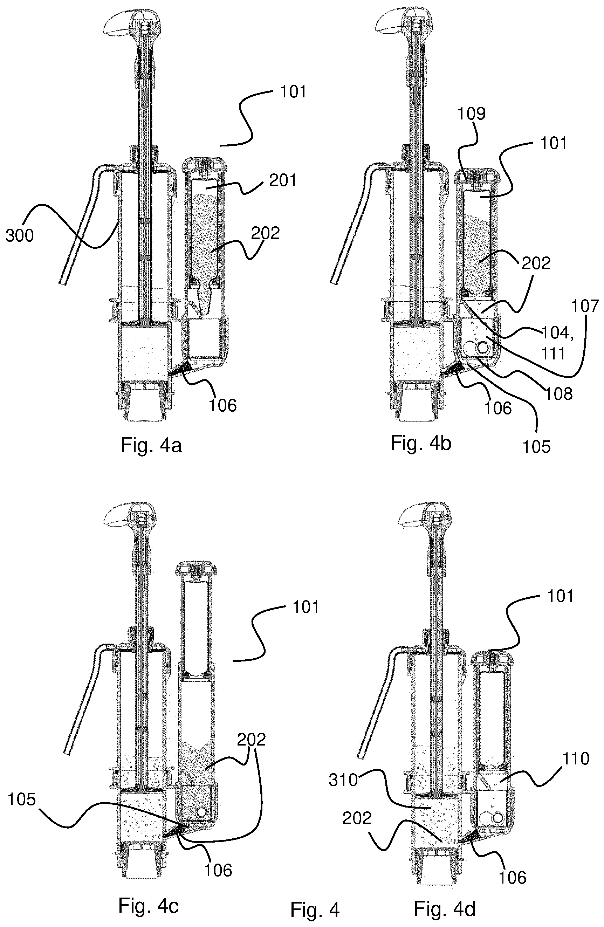

**Figur 4** zeigt mehrere Schritte eines Verfahrens, in welchem eine erfindungsgemäße Vorrichtung zur Herstellung eines PMMA-Knochenzements verwendet wird.

**Figur 4a** zeigt eine erfindungsgemäße Vorrichtung **100** in einem ersten Schritt des Verfahrens. In einem Ampullenbrecher **101** der Vorrichtung ist eine Glasampulle **201** mit einer darin enthaltenen Flüssigkeit **202,** nämlich einer Monomerflüssigkeit zur Herstellung eines PMMA-Knochenzements, aufgenommen. Der Ampullenbrecher **101** ist über einen Kanal **106** mit einer Kartusche **300** verbunden, welche eine Pulverkomponente zur Herstellung eines PMMA-Knochenzements enthält.

**Figur 4b** zeigt eine erfindungsgemäße Vorrichtung **100** in einem zweiten Schritt des Verfahrens. Mithilfe eines Aufbrechelements **104** wird die Glasampulle **201** aufgebrochen, sodass die enthaltene Flüssigkeit **202** in einen ersten Innenraum **110** auslaufen kann. Dies geschieht im hier gezeigten Beispiel mithilfe eines Kolbens **109,** der die Glasampulle **201** gegen einen Vorsprung **111** drückt. Der abgebrochener Ampullenkopf wird in einem Auffangbereich **107** zurückgehalten, während die Flüssigkeit durch ein Sieb **108** in einen Sammelbereich **105** gelangen kann.

**Figur 4c** zeigt eine erfindungsgemäße Vorrichtung **100** in einem dritten Schritt des Verfahrens. Die Flüssigkeit **202** ist durch Gravitationsfluss in den Sammelbereich **105** und den damit fluidleitend verbundenen Kanal **106** gelangt, und wird durch Ausbildung eines Meniskus der Flüssigkeit **202** im Ampullenbrecher **101** zurückgehalten.

**Figur 4d** zeigt eine erfindungsgemäße Vorrichtung **100** in einem vierten Schritt des Verfahrens. Durch Aufbau eines Druckunterschieds zwischen dem ersten Innenraum **110** und dem zweiten Innenraum **310** kann die Flüssigkeit **202** in den zweiten Innenraum **310** gelangen, um sich dort mit der Pulverkomponente zu verbinden.

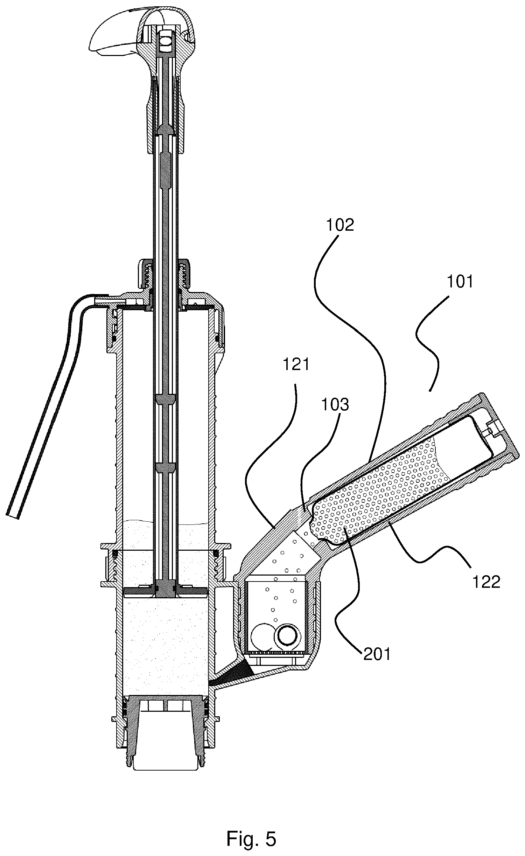

**Figur 5** zeigt eine weitere Ausführungsform einer erfindungsgemäßen Vorrichtung **100.** In der hier gezeigten Ausführungsform ist das Aufbrechelement des Ampullenbrechers **101** in Form eines flexiblen Gehäuseteils **121** in Verbindung mit einem starreren Gehäuseteil **122** ausgestaltet. Das flexible Gehäuseteil **121** und das starre Gehäuseteil **122** sind aneinander angrenzend in dem ersten Gehäuse **102** des Ampullenbrechers **101** angeordnet, sodass sich der Hals **203** einer in der Halterung **103** aufgenommenen Glasampulle **201** im Bereich des Übergangs zwischen dem flexiblen Gehäuseteil **121** und dem starren Gehäuseteil **122** befindet. Durch manuelles Biegen des flexiblen Gehäuseteils **121** gegenüber dem starren Gehäuseteil **122** kann eine Ampulle **201** aufgebrochen werden.

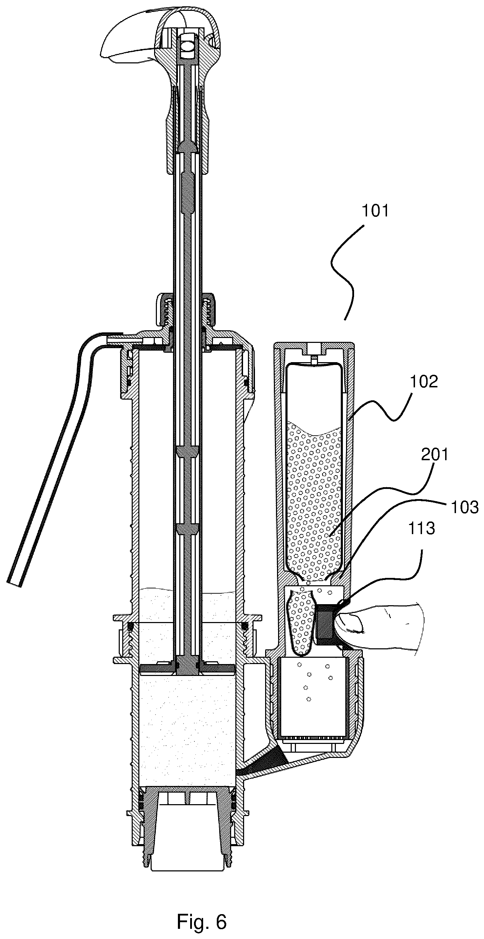

**Figur 6** zeigt eine weitere Ausführungsform einer erfindungsgemäßen Vorrichtung **100.** In der hier gezeigten Ausführungsform ist das Aufbrechelement des Ampullenbrechers **101** in Form einer Taste **113** ausgestaltet, welche von außen in das erste Gehäuse **102** einschiebbar ist. Die Taste **113** kann manuell gegen den Kopf einer in der Halterung **103** angeordneten Glasampulle **201** geschoben und dadurch der Kopf abgebrochen werden, um die Ampulle **201** aufzubrechen. Bevorzugt weist die Taste **113** ein elastisches Element auf, um nach dem Einschieben in das Gehäuse **102** wieder in ihre ursprüngliche Form und Anordnung zurückzukehren.

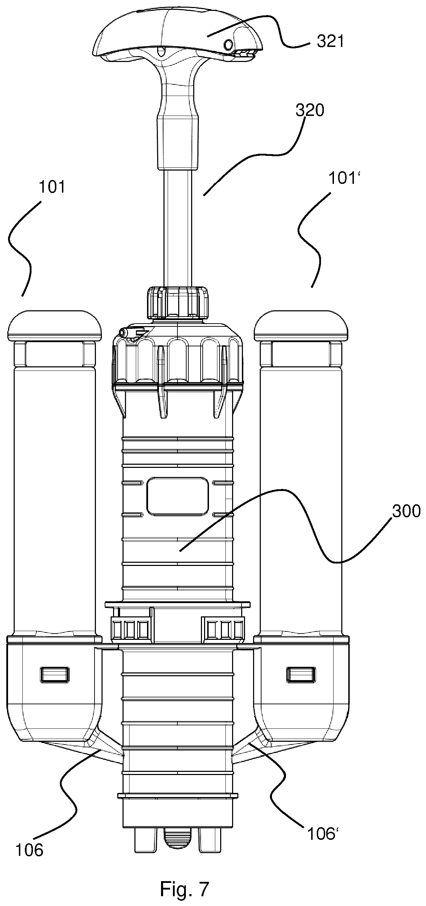

**Figur 7** zeigt eine weitere Ausführungsform einer erfindungsgemäßen Vorrichtung **100.** In der hier gezeigten Ausführungsform weist die Vorrichtung zwei Ampullenbrecher **101, 101'** auf, welche jeweils über einen Kanal **106, 106'** mit derselben Kartusche **300** fluidleitend verbunden sind. In einer solchen Vorrichtung können beispielsweise insgesamt zwei oder insgesamt vier Glasampullen (d. h. zwei Glasampullen pro Ampullenbrecher) gleichzeitig aufgebrochen und in die Kartusche **300** abgegeben werden. Ein Kanal **106** ist zu dem anderen Kanal **106'** spiegelbildlich angeordnet, wobei die beiden Kanäle von oben (d. h. aus Richtung des Griffs **321** des Mischers **320**) betrachtet miteinander einen 180°-Winkel bilden.

### BEZUGSZEICHENLISTE

- 100: Vorrichtung
- 101: Ampullenbrecher
- 102: erstes Gehäuse
- 103: Halterung
- 104: Aufbrechelement
- 105: Sammelbereich (für Flüssigkeit)
- 106: Kanal
- 107: Auffangbereich (für Glasbruch)
- 108: Sieb
- 109: Kolben
- 110: erster Innenraum
- 111: Vorsprung
- 112: Ventil
- 113: Taste
- 114: Einlass des Kanals
- 115: Auslass des Kanals
- 121: flexibler Gehäuseteil
- 122: starrer Gehäuseteil
- 201: Glasampulle
- 202: Flüssigkeit
- 203: Ampullenhals
- 204: Sollbruchstelle
- 300: Kartusche
- 302: zweites Gehäuse
- 303: Vakuumanschluss
- 304: Verschluss
- 305: Durchführung
- 306: Verbindungselement
- 307: erster Teil des zweiten Gehäuses
- 308: zweiter Teil des zweiten Gehäuses
- 310: zweiter Innenraum
- 320: Mischer
- 321: Griff
- 322: Stab
- 323: Scheibe

## Patentansprüche

1. Vorrichtung (100) zur Herstellung eines Knochenzements, aufweisend
- einen Ampullenbrecher (101) mit einem ersten Gehäuse (102), wobei das erste Gehäuse einen ersten Innenraum (110) aufweist,
- eine in dem ersten Gehäuse (102) angeordnete Halterung (103) zur Aufnahme einer Glasampulle (201),
- ein in dem ersten Gehäuse (102) angeordnetes Aufbrechelement (104), das zum Aufbrechen einer in der Halterung (103) aufnehmbaren Glasampulle (201) ausgebildet und eingerichtet ist,
- ein in dem ersten Gehäuse (102) angeordneter Sammelbereich (105), der zur Aufnahme einer Flüssigkeit (202) aus einer aufgebrochenen Glasampulle (201) ausgebildet und eingerichtet ist,
- eine Kartusche (300), die zur Aufnahme einer Pulverkomponente eines Knochenzements eingerichtet ist, wobei die Kartusche (300) ein zweites Gehäuse (302) mit einem zweiten Innenraum (310) aufweist,
- einen fluidleitenden Kanal (106) zur Abgabe einer Flüssigkeit (202) aus dem Sammelbereich (105) in den zweiten Innenraum (310), **dadurch gekennzeichnet, dass**
der fluidleitende Kanal (106) angeordnet und aufgrund eines geringen Innendurchmessers eingerichtet ist, die Flüssigkeit (202), durch die Ausbildung eines Meniskus der in dem Kanal (106) aufgenommenen Flüssigkeit (202), in dem Sammelbereich (105) zurückzuhalten, und die Flüssigkeit an den zweiten Innenraum (310) abzugeben, sobald zwischen dem Sammelbereich (105) und dem zweiten Innenraum (310) ein Druckunterschied anliegt.

2. Vorrichtung nach Anspruch 1, weiterhin aufweisend einen Auffangbereich (107), der in dem ersten Gehäuse (102) angeordnet und zur Aufnahme eines durch Wirkung des Aufbrechelements (104) abgebrochenen Teils einer Glasampulle (201) eingerichtet ist.

3. Vorrichtung nach Anspruch 2, weiterhin aufweisend ein Sieb (108), welches angrenzend an den Auffangbereich (107) angeordnet ist, und eingerichtet ist, Bruchstücke aus einer in der Vorrichtung aufgebrochenen Glasampulle (201) im Ampullenbrecher (101) zurückzuhalten.

4. Vorrichtung nach einem der vorangehenden Ansprüche, wobei der fluidleitende Kanal (106) in einem Winkel α von 60° bis 90° zu dem zweiten Gehäuse (302) angeordnet ist.

5. Vorrichtung nach einem der vorangehenden Ansprüche, wobei der fluidleitende Kanal (106) zumindest abschnittsweise einen Innendurchmesser von weniger als 5 mm, bevorzugt weniger als 4 mm oder weiter bevorzugt weniger als 3 mm aufweist.

6. Vorrichtung nach einem der vorangehenden Ansprüche, wobei eine Glasampulle (201) kraftschlüssig, formschlüssig oder stoffschlüssig in der Halterung (103) befestigt oder fixierbar ist, bevorzugt durch Kleben oder Klemmen befestigt oder fixierbar ist.

7. Vorrichtung nach Anspruch 6, wobei die Glasampulle (201) einen Ampullenhals (203) mit einer ringförmigen Sollbruchstelle (204) aufweist, wobei der Innendurchmesser der Sollbruchstelle (204) bevorzugt 4 bis 7 mm beträgt.

8. Vorrichtung nach einem der vorangehenden Ansprüche, wobei die Kartusche (300) weiterhin eine Pulverkomponente zur Zubereitung eines Knochenzements enthält, wobei die Pulverkomponente bevorzugt in dem zweiten Innenraum (310) des zweiten Gehäuses (302) angeordnet ist.

9. Vorrichtung nach einem der vorangehenden Ansprüche, wobei der Ampullenbrecher (101) zum gleichzeitigen Aufbrechen und Abgeben von Flüssigkeit (202) aus zwei Glasampullen (201) an die Kartusche (300) ausgebildet und eingerichtet ist.

10. Vorrichtung nach einem der vorangehenden Ansprüche, wobei das Aufbrechelement (104) einen in dem ersten Gehäuse (102) beweglich angeordneten Kolben (109) und einen Vorsprung (111) aufweist, wobei der Kolben (109) ausgebildet und eingerichtet ist, eine in der Halterung (103) aufnehmbare Glasampulle (201) mit einer Flüssigkeit (202) durch Druck der Glasampulle (201) gegen den Vorsprung (111) aufzubrechen, und die Flüssigkeit (202) aus der Glasampulle (201) durch den Kanal (106) aus dem ersten Gehäuse (101) in das zweite Gehäuse (302) zu pumpen.

11. Vorrichtung nach Anspruch 10, wobei der Ampullenbrecher (101) weiterhin ein Ventil (112) aufweist, welches eingerichtet ist, Luft von außen in das erste Gehäuse (102) einströmen zu lassen, um anschließend durch Druck des Kolbens (109) auf die in das erste Gehäuse (102) eingeströmte Luft eine Flüssigkeit (202) aus einer in der Vorrichtung aufgebrochenen Glasampulle (201) durch den Kanal (106) aus dem ersten Gehäuse (101) in das zweite Gehäuse (302) zu pumpen.

12. Vorrichtung nach Anspruch 11, wobei das Ventil (106) ein Einwegeventil, bevorzugt ein Kugelventil, Lippenventil oder Plattenventil ist.

13. Vorrichtung nach einem der vorangehenden Ansprüche 10 bis 12, wobei der Ampullenbrecher (101) ausgebildet und eingerichtet ist, mit einem einzigen Hub des Kolbens (105) den gesamten Inhalt einer in der Halterung (102) aufgenommenen Glasampulle (201) durch den Kanal (106) aus dem ersten Gehäuse (101) in das zweite Gehäuse (302) zu pumpen.

14. Vorrichtung nach einem der Ansprüche 1 bis 9, wobei das Aufbrechelement (104) eine von außen in das erste Gehäuse (102) einschiebbare Taste (113) aufweist, um eine in der Halterung (103) aufnehmbare Glasampulle (201) durch Einschieben der Taste (113) in das erste Gehäuse (102) aufzubrechen, wobei die Taste (113) bevorzugt eingerichtet ist, nach Einschieben der Taste (113) selbsttätig in ihre ursprüngliche Position zurückzukehren.

15. Vorrichtung nach einem der Ansprüche 1 bis 9, wobei das erste Gehäuse (102) des Ampullenbrechers (101) zumindest teilweise biegsam ist, um eine in der Halterung (103) aufnehmbare Glasampulle (201) durch Biegen des ersten Gehäuses (102) aufzubrechen.

## Claims

1. A device (100) for producing a bone cement, comprising
- an ampule breaker (101) having a first housing (102), the first housing comprising a first interior (110),
- a holder (103) arranged in the first housing (102) for receiving a glass ampule (201),
- a breaking element (104) which is arranged in the first housing (102) and is designed and configured to break open a glass ampule (201) which can be received in the holder (103),
- a collection region (105) which is arranged in the first housing (102) and is designed and configured to receive a liquid (202) from a broken-open glass ampule (201),
- a cartridge (300) configured to receive a powder component of a bone cement, the cartridge (300) comprising a second housing (302) having a second interior (310),
- a fluid-conducting channel (106) for discharging a liquid (202) from the collection region (105) into the second interior (310), **characterized in that**
the fluid-conducting channel (106) is arranged and, due to a small inner diameter, is designed to retain the liquid (202) in the collection region (105) by forming a meniscus of the liquid (202) received in the channel (106), and to discharge the liquid to the second interior (310) as soon as a pressure difference exists between the collection region (105) and the second interior (310).

2. The device according to claim 1, further comprising a catchment region (107) which is arranged in the first housing (102) and is configured to receive a portion of a glass ampule (201) that is broken off by the action of the breaking element (104).

3. The device according to claim 2, further comprising a screen (108) which is arranged adjacent to the catchment region (107) and is configured to retain fragments from a glass ampule (201) broken open in the device in the ampule breaker (101).

4. The device according to any of the preceding claims, wherein the fluid-conducting channel (106) is arranged at an angle α of 60° to 90° to the second housing (302).

5. The device according to any of the preceding claims, wherein the fluid-conducting channel (106) has an inner diameter of less than 5 mm, preferably less than 4 mm or more preferably less than 3 mm, at least in portions.

6. The device according to any of the preceding claims, wherein a glass ampule (201) is fastened or fixable in the holder (103) in a force-fitting, form-fitting or integrally bonded manner, and preferably is fastened or fixable by adhesive bonding or clamping.

7. The device according to claim 6, wherein the glass ampule (201) comprises an ampule neck (203) having an annular predetermined breaking point (204), wherein the inner diameter of the predetermined breaking point (204) is preferably 4 to 7 mm.

8. The device according to any of the preceding claims, wherein the cartridge (300) further contains a powder component for preparing a bone cement, wherein the powder component is preferably arranged in the second interior (310) of the second housing (302).

9. The device according to any of the preceding claims, wherein the ampule breaker (101) is designed and configured for simultaneously breaking open and dispensing liquid (202) from two glass ampules (201) to the cartridge (300).

10. The device according to any of the preceding claims, wherein the breaking element (104) comprises a plunger (109) arranged movably in the first housing (102), and a projection (111), wherein the plunger (109) is designed and configured to break open a glass ampule (201) with a liquid (202), which glass ampule can be received in the holder (103), by pressure of the glass ampule (201) against the projection (111), and to pump the liquid (202) from the glass ampule (201) through the channel (106) out of the first housing (101) into the second housing (302).

11. The device according to claim 10, wherein the ampule breaker (101) further comprises a valve (112) which is configured to allow air to flow into the first housing (102) from the outside in order to subsequently pump a liquid (202) from a glass ampule (201) broken open in the device through the channel (106) out of the first housing (101) into the second housing (302) by pressure of the plunger (109) onto the air flowing into the first housing (102).

12. The device according to claim 11, wherein the valve (106) is a one-way valve, preferably a ball valve, lip valve, or plate valve.

13. The device according to any of the preceding claims 10 to 12, wherein the ampule breaker (101) is designed and configured to pump the entire contents of a glass ampule (201) received in the holder (102) through the channel (106) out of the first housing (101) into the second housing (302) with a single stroke of the plunger (105).

14. The device according to any of claims 1 to 9, wherein the breaking element (104) comprises a button (113) which can be pushed into the first housing (102) from the outside in order to break open a glass ampule (201), which can be received in the holder (103), by pushing the button (113) into the first housing (102), wherein the button (113) is preferably configured to automatically return to its original position after the button (113) has been pushed in.

15. The device according to any of claims 1 to 9, wherein the first housing (102) of the ampule breaker (101) is at least partially flexible in order to break open a glass ampule (201) that can be received in the holder (103) by bending the first housing (102).

## Revendications

1. Dispositif (100) pour la fabrication d'un ciment osseux, présentant
- un moyen de bris d'ampoule (101) comportant un premier boîtier (102), dans lequel le premier boîtier présente un premier espace intérieur (110),
- un support (103) disposé dans le premier boîtier (102) pour la réception d'une ampoule en verre (201),
- un élément de bris (104) disposé dans le premier boîtier (102) et réalisé et conçu pour le bris d'une ampoule en verre (201) pouvant être reçue dans le support (103),
- une zone de collecte (105) disposée dans le premier boîtier (102) et réalisée et conçue pour la réception d'un liquide (202) provenant d'une ampoule en verre (201) brisée,
- une cartouche (300) qui est conçue pour la réception d'un composant en poudre d'un ciment osseux, dans lequel la cartouche (300) présente un second boîtier (302) comportant un second espace intérieur (310),
- un canal (106) de conduction de fluide pour la distribution d'un liquide (202) depuis la zone de collecte (105) dans le second espace intérieur (310), **caractérisé en ce que** le canal (106) de conduction de fluide est disposé et, en raison d'un faible diamètre intérieur, conçu pour retenir le liquide (202) dans la zone de collecte (105) par la formation d'un ménisque du liquide (202) reçu dans le canal (106), et pour distribuer le liquide dans le second espace intérieur (310) dès qu'une différence de pression est appliquée entre la zone de collecte (105) et le second espace intérieur (310).

2. Dispositif selon la revendication 1, présentant en outre une zone de récupération (107) qui est disposée dans le premier boîtier (102) et est conçue pour la réception d'une partie d'une ampoule en verre (201) brisée par l'action de l'élément de bris (104).

3. Dispositif selon la revendication 2, présentant en outre un tamis (108) qui est disposé de manière à être adjacent à la zone de récupération (107) et est conçu pour retenir des fragments d'une ampoule en verre (201) brisée dans le dispositif dans le moyen de bris d'ampoule (101).

4. Dispositif selon l'une des revendications précédentes, dans lequel le canal de conduction de fluide (106) est disposé selon un angle α allant de 60° à 90° par rapport au second boîtier (302).

5. Dispositif selon l'une des revendications précédentes, dans lequel le canal (106) de conduction de fluide présente, au moins dans certaines sections, un diamètre intérieur inférieur à 5 mm, de préférence inférieur à 4 mm ou plus préférablement inférieur à 3 mm.

6. Dispositif selon l'une des revendications précédentes, dans lequel une ampoule en verre (201) est fixée ou peut être fixée dans le support (103) par adhérence, par complémentarité de forme ou par liaison de matière, de préférence est fixée ou peut être fixée par collage ou par serrage.

7. Dispositif selon la revendication 6, dans lequel l'ampoule en verre (201) présente un col d'ampoule (203) comportant un point de rupture (204) annulaire, dans lequel le diamètre intérieur du point de rupture (204) va de préférence de 4 à 7 mm.

8. Dispositif selon l'une des revendications précédentes, dans lequel la cartouche (300) contient en outre un composant en poudre pour la préparation d'un ciment osseux, dans lequel le composant en poudre est de préférence disposé dans le second espace intérieur (310) du second boîtier (302).

9. Dispositif selon l'une des revendications précédentes, dans lequel le moyen de bris d'ampoule (101) est réalisé et conçu pour le bris et la distribution simultanés du liquide (202) de deux ampoules en verre (201) à la cartouche (300).

10. Dispositif selon l'une des revendications précédentes, dans lequel l'élément de bris (104) présente un piston (109) disposé de manière à être mobile dans le premier boîtier (102) et une saillie (111), dans lequel le piston (109) est réalisé et conçu pour briser une ampoule en verre (201) pouvant être reçue dans le support (103) et comportant un liquide (202) en pressant l'ampoule en verre (201) contre la saillie (111), et pour pomper le liquide (202) provenant de l'ampoule en verre (201) du premier boîtier (101) dans le second boîtier (302) par l'intermédiaire du canal (106).

11. Dispositif selon la revendication 10, dans lequel le moyen de bris d'ampoule (101) présente en outre une soupape (112) qui est conçue pour permettre à de l'air d'entrer dans le premier boîtier (102) depuis l'extérieur afin d'ensuite pomper un liquide (202) provenant d'une ampoule en verre (201) brisée dans le dispositif du premier boîtier (101) dans le second boîtier (302) par l'intermédiaire du canal (106), par pression du piston (109) sur l'air entré dans le premier boîtier (102).

12. Dispositif selon la revendication 11, dans lequel la soupape (106) est une soupape unidirectionnelle, de préférence une soupape à bille, une soupape à lèvre ou une soupape à plaque.

13. Dispositif selon l'une des revendications 10 à 12 précédentes, dans lequel le moyen de bris d'ampoule (101) est réalisé et conçu pour pomper, en une seule course du piston (105), la totalité du contenu d'une ampoule en verre (201) reçue dans le support (102) du premier boîtier (101) dans le second boîtier (302) par l'intermédiaire du canal (106).

14. Dispositif selon l'une des revendications 1 à 9, dans lequel l'élément de bris (104) présente un bouton (113) pouvant être inséré dans le premier boîtier (102) depuis l'extérieur afin de briser une ampoule en verre (201) pouvant être reçue dans le support (103) en insérant le bouton (113) dans le premier boîtier (102), dans lequel le bouton (113) est de préférence conçu pour revenir automatiquement dans sa position initiale après l'insertion du bouton (113).

15. Dispositif selon l'une des revendications 1 à 9, dans lequel le premier boîtier (102) du moyen de bris d'ampoule (101) est au moins partiellement flexible pour briser une ampoule en verre (201) pouvant être reçue dans le support (103) en pliant le premier boîtier (102).
